Europäisches Patentamt

**European Patent Office**

Office européen des brevets

(11) Publication number: **0 243 547 B1**

# EUROPEAN PATENT SPECIFICATION

(45) Date of publication of patent specification: **24.07.91**   (51) Int. Cl.⁵: **A61M 1/28**, A61M 1/30

(21) Application number: **86303335.3**

(22) Date of filing: **01.05.86**

(54) **Continuous flow peritoneal dialysis apparatus.**

(43) Date of publication of application:
**04.11.87 Bulletin 87/45**

(45) Publication of the grant of the patent:
**24.07.91 Bulletin 91/30**

(84) Designated Contracting States:
**AT BE CH DE FR GB IT LI LU NL SE**

(56) References cited:
EP-A- 0 088 900          DE-A- 2 149 040
FR-A- 2 371 931          US-A- 3 545 438
US-A- 3 709 222          US-A- 4 338 190

(73) Proprietor: **Peabody, Alan M.**
**102 Meadow Creek Court**
**Greer South Carolina 29651(US)**

(72) Inventor: **Peabody, Alan M.**
**102 Meadow Creek Court**
**Greer South Carolina 29651(US)**

(74) Representative: **Purvis, William Michael**
**Cameron et al**
**D. Young & Co. 10 Staple Inn**
**London WC1V 7RD(GB)**

## Description

The invention relates to peritoneal dialysis apparatus for purifying blood by supply of a dialysate and exchange across the peritoneal membrane of a patient.

Heretofore, artificial kidney users have relied basically on two processes for purifying the blood. Hemodialysis involves the circulation of blood through a dialysis machine in which an exchange of toxic metabolites takes place across an artificial membrane outside of the patient's body. This process requires the assistance of trained personnel and subjects the patient to dangers of mechanical malfunction due to the fact that blood vessels are involved.

Peritoneal dialysis involves the infusion of a sterile dialysate into the peritoneal cavity and after absorbing waste metabolites, the dialysate is discarded. The process is then repeated until the level of metabolytes is reduced to a desired level. This method is commonly referred to as the "Batch" method due to the fact that multiple one or two litre bottles or bags of fresh dialysate solution are utilized which require multiple connections to be made to a catheter inserted in the peritoneal cavity during the dialysis process. The multiple connections made during the course of the dialysis has been thought to be a major cause of the high instance of peritonitis.

Continuous ambulatory peritoneal dialysis offers continuous peritoneal dialysis while still allowing the patient some off time. However, the continuous ambulatory peritoneal dialysis must be done in the absence of a machine and multiple bottles or bags of dialysis must be infused daily. Thus, multiple infusions per day requires that multiple connections of bags or bottles to the peritoneal catheter be made. The production of bulk sterile dialysis for the peritoneal process has not been shown to be practical for large scale application particularly for home dialysis.

United States Patent US-A-4 311 587 seeks to avoid some of the above problems with peritoneal dialysis by providing a sub-micron filter on line with the fresh dialysate to prevent peritoneal contamination. The system is perambulatory and the bag of dialysate is worn by the patient. The bag may be pressurized by numerous methods and is connected only to the inflow side of the filter. The outflow port of the filter is connected on the other side of the filter so that no peritoneal contaminating source is connected directly to the peritoneal cavity. The system is still basically a batch type system in that multiple bottles or bags of dialysate must be connected to the filter even though direct connection to the peritoneal catheter is not required.

United States Patent US-A-4 338 190, on which the preamble of the claim 1 is based, discloses a system and process which attempts to avoid the batch process method utilized heretofore in peritoneal dialysis wherein a closed loop peritoneal circuit is provided having a selective membrane across which toxic metabolites are exchanged. A solution is passed on the other side of the selective membrane for maintaining the original concentration of sugar and salt in the peritoneal fluid as the toxic metabolites pass the separator membrane. A double peritoneal catheter provides for the inflow and outflow of the peritoneal fluid. However, the peritoneal fluid is constantly recirculated through the peritoneal cavity and the efficiency becomes reduced slightly because of residual toxins which are put back into the peritoneal cavity. The selective membrane is an expensive disposable item which means that the cost of operating the system is high unless the membrane is recleaned. Pumping the peritoneal fluid through the peritoneal cavity is required making it difficult to ensure that the patient stays properly distended during the dialysis process. If the peritoneal membrane is not fully distended, it becomes convoluted around the intestines and pockets are formed where the peritoneal fluid can hide. Incomplete circulation then results with decreased efficiency of dialysis. There is no control over the level of the peritoneal fluid in the peritoneal circuit and no way of replenishing the peritoneal fluid should the circuit run low on fluid or run dry.

French Patent 2 371 931 discloses an intermittent system wherein dialysis is effected in three phases. In a first phase a controlled volume of fluid is pumped into the peritoneal cavity of a patient; in a second phase there is diffusion by allowing the fluid to dwell in the peritoneal cavity for a set period of time; and in the third phase there is drainage in that the exact amount of fluid pumped into the cavity is allowed to drain out. There may also be secondary drainage whereby ultra-filtrated fluid is removed.

According to the invention there is provided Peritoneal dialysis apparatus comprising:
a source of sterile dialysis fluid;
an inflow line for connection to the source and to a peritoneal catheter implantable in the peritoneal cavity of a patient; and
an outflow line for connection to the peritoneal catheter; whereby a generally continuous flow of sterile dialysis fluid is produced and caused to flow though the peritoneal cavity;
characterised by valve means for controlling the flow of dialysis fluid in the inflow and outflow lines thereby to control the volume of dialysis fluid in the peritoneal cavity of the patient;
disposal means connected to the outflow line for disposing of the outflow of dialysis fluid and establishing a single-pass open circuit through the peritoneal cavity for dialysis;

flow monitoring means for sensing a function of the volume and amount of dialysis fluid in the peritoneal cavity; and

control means controlling the valve means in response to the flow monitoring means so that flow of the dialysis fluid through the peritoneal cavity is controlled in a manner such that a desired amount of fluid exists in the peritoneal cavity for proper dialysis.

Such peritoneal dialysis apparatus can avoid the inherent problems and dangers of a batch type peritoneal dialysis system and can have a high rate of dialysate exchange providing increased dialysis efficiency.

The peritoneal dialysis process, which can be carried out with the apparatus of the invention, can have a high rate of dialysate exchange and dialysis efficiency in which the danger of peritoneal infection is minimized.

The apparatus can monitor the pressure and volume of dialysate in the patient's peritoneal membrane to ensure for proper distention of the membrane at all times.

Thus a continuous flow of sterile dialysis fluid is produced and caused to flow through the peritoneal cavity of the patient in a single-pass open-circuit. The exchange of toxic metabolites occurs across the patient's peritoneal membrane and the residual dialysis solution is drained away after leaving the patient's cavity. A gravity fed system is utilized avoiding the pumping of the fluid through the cavity. A by-pass valve is utilized in combination with a flow monitor to adjust the inflow of sterile dialysis fluid going into the peritoneal cavity and make the inflow equal to the flow coming out of the cavity so that a predetermined volume of fluid in the cavity is maintained at all times. A pressure monitor is utilized to sense the pressure of the fluid in the cavity to make sure that the membrane is not over-distended or under-distended for efficient and comfortable peritoneal dialysis. The pressure monitor controls an inflow/outflow directional valve. If the pressure becomes excessive causing over -distention, the inflow is cut-off or throttled and the outflow is left opened or opened further as need be to control or relieve pressure. If the pressure drops, causing under-distention and convolutions, the directional valve throttles outflow and opens inflow as needed. The continuous flow of peritoneal dialysis fluid provides a high rate of toxic metabolite exchange as the fluid flows in a single pass through the cavity. In this manner, dialysis for six to eight hours approximately three times a week is sufficient for peritoneal dialysis.

The invention is diagrammatically illustrated by way of example with reference to the accompanying drawings, in which:-

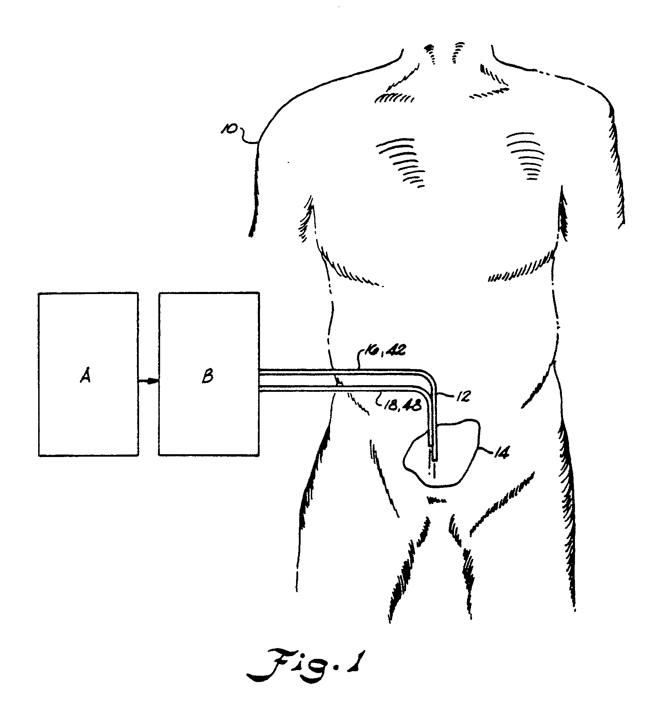

Figure 1 is a schematic elevation illustrating a continuous flow peritoneal dialysis apparatus according to the invention coupled to a patient implant;

Figure 2 is a schematic view illustrating the apparatus of Figure 1;

Figure 3 is a schematic illustration of another embodiment of apparatus according to the invention; and

Figure 3A is a schematic illustration of a still further embodiment of apparatus according to the invention.

As currently practised, peritoneal dialysis is about one-fifth as efficient as hemodialysis in the removal of solute from the body. Previous studies have demonstrated that the efficiency of peritoneal dialysis can be made to approximate to that of hemodialysis if the rate of dialysate exchange is raised to ten litres or more per hour. At present, the usual rate of exchange of peritoneal dialysis is approximately two litres every four hours. It is desirable therefore continuously to produce moderate quantities of sterile dialysis fluid for peritoneal dialysis and provide apparatus for passing a continuous-flow of the sterile dialysis fluid through the peritoneal cavity of the patient in an open-circuit.

Referring now in more detail to the drawings, a patient is illustrated at 10 having a continuous flow peritoneal dialysis catheter 12 implanted in the peritoneal membrane 14 of the patient. As shown schematically, the implanted catheter is connected to suitable tubing 16 and 18 which is then, in turn, connected to a continuous flow peritoneal dialysis system which includes a fluid supply system A and a fluid delivery control system B. The fluid delivery control system includes a single-pass circuit through the peritoneal cavity of the patient.

The fluid supply system A includes a particle filter 20 to which a flow of tap water is directed. Water passing through the particle filter next goes to a reverse osmosis unit 22. From the reverse osmosis unit the water flows through a carbon filter 24 which takes out the chlorine in the water. From the carbon filter 24 the water flows into a conventional proportioning machine 26 which takes a 34:1 concentrate solution of non-sterile glucose and electrolytes from a supply 27 and mixes it with a proper amount of the purified water coming from the carbon filter 24.

The dialysis solution from the proportioning and mixing unit 26 then passes to a micron particle filter 28 which separates out the non-dissolved particulate matter. Quite often the concentrate being mixed in the proportioning and mixing unit 26 includes some non-dissolved particulate matter. The micron filter 28 is preferably a one (1) micron filter which has an opening sufficient to remove the non-dissolved particulate matter. Both the Milipore and Gilman Manufacturing Companies manufacture a suitable micron filter. The fluid supply system A provides a properly mixed dialysis solution or dialysate which is non-sterile. The non-sterile fluid from the micron filter 28 is delivered to the continuous fluid delivery control system 13. At the outset, the non-sterile dialysis solution flows through a bacterial filter 30 which sterilizes the dialysis solution. The bacterial filter 30 is a conventional high volume bacterial filter which removes all the bacteria to provide a sterile dialysis solution. The fluid then flows to a head vessel 32 which provides a gravity feed means for feeding the sterilized dialysis fluid. The proportioning and mixing unit 26 includes a pump which is sufficient to pump the dialysis fluid into the head vessel.

A by-pass valve 34 is connected to the outflow of the head vessel 32. The by-pass valve 34 provides a means for controlling the fluid level in the vessel and preventing the head vessel from overflowing. In the event that the head vessel begins to overflow or contains excess sterilized fluid coming from the fluid supply system A, the by-pass valve dumps the excess fluid to a storage container 36. It has been found that it is easier to dump the dialysate rather than start and stop the proportioning and mixing unit 26. A level monitor 38 provides a means for sensing the level of the dialysate fluid in the head vessel 32 and controlling the by-pass valve 34 in response to the fluid level.

Switching means may also be provided responsive to the level monitor 38 to cut off the proportioning and mixing unit 26 in the event that the by-pass valve does not operate correctly and the fluid backs up excessively.

An inflow/outflow directional valve 40 is connected to the by-pass valve 34 for receiving sterile dialysis fluid from the head vessel 32. The by-pass valve 34 delivers dialysis fluid to the directional valve 40 in an amount depending on the degree to which fluid is being by-passed to the storage container 36. The by-pass valve 34 is controlled in a manner which will be more fully set forth hereinafter to vary the amount which is delivered to the directional valve 40. The by-pass valve 34 may be a throttling valve so that it has stepless variability for by-passing fluid from the line to the directional valve 40.

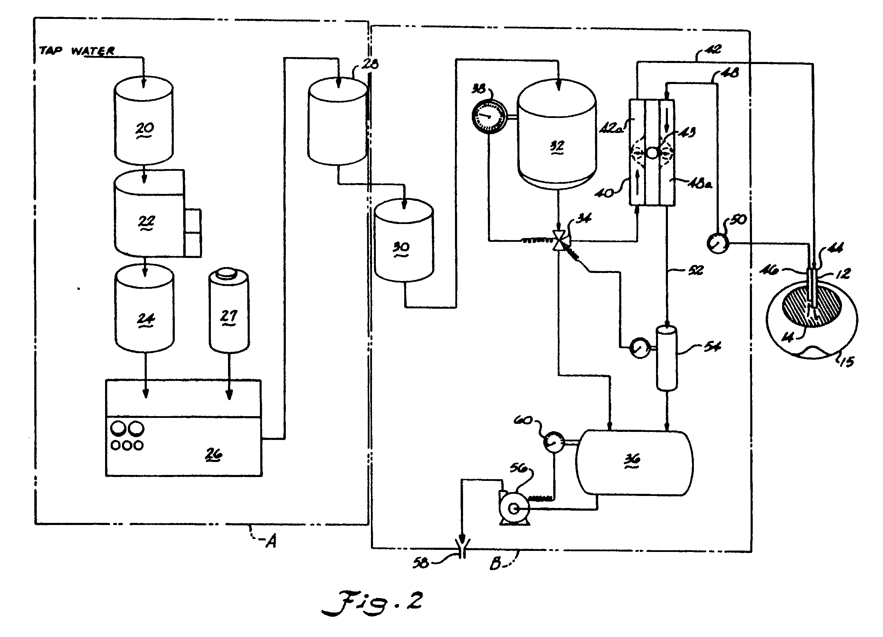

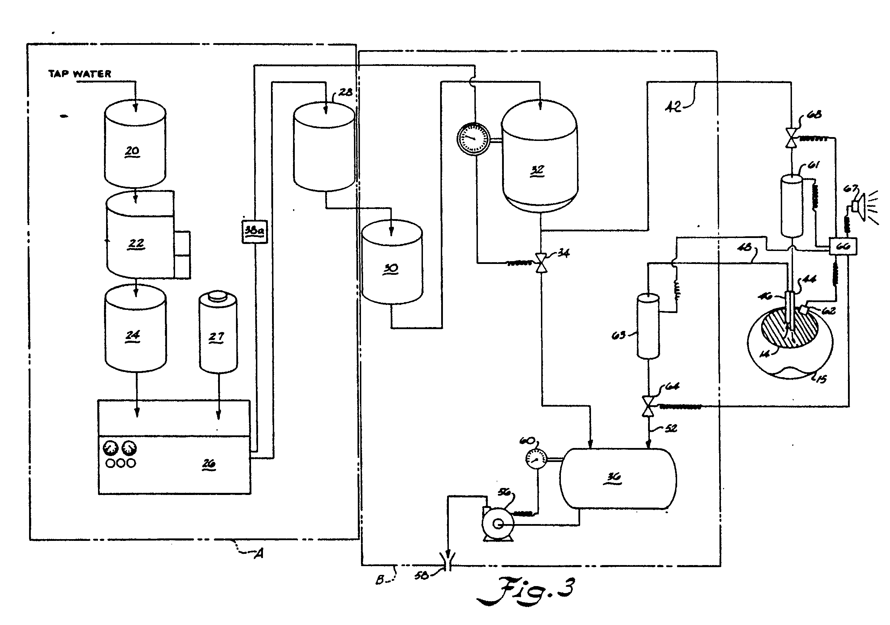

An inflow line 42 (tubing 16) is connected to the directional valve 40 and to an inflow catheter 44 of the double peritoneal catheter 12. The dialysis fluid leaves the peritoneal cavity 14 through an outflow catheter 46 by way of an outflow line 48 (tubing 18) which is controlled by and goes through the directional valve 40. There is a pressure monitoring means 50 connected in the outflow line 48 for sensing the pressure of the fluid in the line and in the peritoneal cavity. The pressure monitor 50 controls the directional valve 40 to maintain a predetermined pressure or pressure range in the peritoneal cavity such that the membrane is properly distended at all times. For this purpose, the directional valve 40 is preferably a double flow valve to provide flows into the catheter and out of the catheter depending on the sensing by the pressure monitor 50. A separate inflow line and outflow line must be maintained through the valve 40 to ensure sterilization of the inflowing dialysis fluid. A modified single needle hemodialysis control valve manufactured by Vernitron corporation is suitable. The modified valve is shown schematically in Figure 2 including an inflow passage 42a and outflow passage 48a controlled by a displaceable needle valve 43 which can be moved partially or fully to occlude either passage in response to abdominal pressure. Normally such a valve is pressure controlled but has only a single outlet line.

In the case where there is excessive pressure in the peritoneal cavity 14 causing the membrane to be overly distended and excessive force to be exerted on the abdominal contents causing discomfort to the patient, the pressure monitor 50 will send a signal (electrical or mechanical) to the valve 40 to stop the inflow of the dialysis fluid while maintaining an outflow of the dialysis fluid so that the pressure is relieved. In this case, in the preferred embodiment, the needle 43 will occlude the inflow passage 42a, completely in lieu of throttling, while the outflow passage 48a will remain open to relieve pressure. If the pressure should drop from that normally desired in the cavity, causing under-distention of the membrane and forming of convolutions in which the dialysis fluid may hide thus decreasing the efficiency of the dialysis process, the outflow of dialysis fluid will be cut off by moving the needle valve 43 proportionately to occlude the passage 48a and the inflow will be continued so that the pressure is increased as desired. In either case, once the pressure is brought back to a desired value, the inflow and outflow of the dialysis fluid will be brought back to being equal. The pressure range which is normally desired in the abdominal cavity during dialysis is about 150 to 250cm/water. Any conventional commercially available pressure monitor operable in this range is suitable for the pressure monitor 50. The control of the valve 40 in response to pressure may be effected by any suitable means as is well within the purview of one skilled in the art. For example, electrical control may be effected with a single integrated circuit receiving the pressure signals and electrically controlling the needle valve 43.

A flow monitor 54 is inserted in an outflow line 52 which goes from the directional valve 40 to the storage tank 36. The flow monitor 54 monitors the outflow and adjusts the flow through the by -pass valve 34 to the directional valve 40 so that the inflow provided by the by-pass valve 34 to the directional valve 40, is the same as the outflow from the peritoneal catheter. When the directional valve 40 is in its normal setting, with the needle valve 43 in its neutral position, any change in the amount by-passed to the storage tank 36 by the valve 34 as controlled by the monitor 54 will directly influence the amount delivered through the inflow passage 42a. Hence, should the outflow drop, for example, the valve 34 will be opened to by-pass more fluid and hence deliver less to the valve 40 thus matching the inflow to the decreased outflow. At the same time, the pressure monitor 50 keeps check on the pressure inside the peritoneal cavity. The flow monitor 54 may be any suitable sensor such as an ultrasonic flow monitor which is conventionally used.

The valve 40, as controlled by the pressure monitor 50, and the valve 34, as controlled by the flow monitor 54, provide flow control means for ensuring the proper volume and pressure of dialysis fluid in the peritoneal cavity. Normally, the needle valve 43 will be in its neutral position and the flow monitor will act to control the inflow in response to outflow by diverting fluid through the valve 34 instead of through the valve 40. However, if in so regulating the inflow to equal outflow, the above pressure limits are exceeded, the monitor 50 can effect relief by controlling the valve 40 as described above. The head vessel monitor 38 is independent of the pressure and flow regulating system.

The directional valve 40 could also be a throttling valve instead of an on/off valve. In such a case, control of the by-pass valve 34 may not be necessary.

The inflow line 42 and the outflow line 48 are connected to the inflow catheter 44 and the outflow catheter 46 by conventional means. This connection is made at the beginning of dialysis and disconnected at the end in a conventional manner.

A drain pump 56 drains the dialysis fluid in the drain storage container 36 whenever a high level is reached. The fluid is drained into a drain 58 where it is drained away. For this purpose, there is a fluid level detector 60 provided which controls the operation of the pump 56. The aforedescribed drain apparatus provides a disposal means for the dialysis fluid which completes the open, single-pass circuit.

The pressure monitor 50 provides a means for sensing the pressure in the abdomen of the patient. The abdomen has a particular pressure, for example, at two litres, that is comfortable. If, for example, one litre is accumulated in the abdominal area, the inner abdominal area is caused to expand by fifty percent and this results in a large degree of discomfort to the patient. In the event the patient becomes overdistended, the pressure monitor will sense a decrease in pressure. In this event, the pressure monitor will control the valve 40 which allows additional fluid to enter the cavity to bring the pressure back up to normal. In the event that the cavity becomes underdistended, the pressure will increase in pressure to cause the fluid to cut down the inflow and let the outflow of fluid continue until the pressure has stabilized.

Thus it can be seen that the invention can provide continuous flow peritoneal dialysis apparatus supplying a single-open circuit through the patient's peritoneal cavity. A sterilized peritoneal dialysis fluid is provided which is delivered through the cavity in such a manner that the volume and pressure of the fluid are controlled so that proper distention of the membrane occurs for patient comfort and dialysis efficiency. The high rate of fluid flow through the peritoneal cavity is advantageous in that the high rate of circulation increases dialysis efficiency.

Pumping, and its inherent problems, through the cavity is eliminated by a gravity feed system and a drainage tank. By providing a single pass circuit, no recirculation of the dialysis fluid through the membrane occurs reducing any likelihood of residual toxins being recirculated through the peritoneal cavity. By monitoring the outflow of the dialysis fluid from the peritoneal cavity and pressure therein, the membrane is spread out to an efficient condition during dialysis so that no pockets or other hiding places for fluid are formed to cut down on the efficiency of the dialysis process.

It is understood that the line from the bacterial filter 30 to the head vessel 32 will be a disposable presterilized line of tubing. The head vessel 32 will be provided with a sterilized liner which is disposable and connected to presterilized tubing which connects the interior of the liner to the by-pass valve 34. In practice, the by-pass valve 34 will have three legs of disposable tubing in a Y-shape. The lower Y-leg of the disposable valve will be connected to the disposal storage tank 36. The actuator of the valve 34 will be external of the sterilized valve passages and will act upon the Y -leg of the valve passages leading to the disposal tank 36 to occlude the passage more or less in order to divert more or less fluid from the inflow/outflow directional valve 40. The inflow/outflow directional valve 40 will be a unit where the valve element 43 is external of tubing which passes through the passages 42a and 48a. The fluid passing through the tubing goes through the valve passages out of contact with any part of the valve so that the valve never has to be sterilized itself. Likewise, the tubing going through the valve passage 42a and connected to the inflow catheter 44 will be disposable. The tubing 48 coming from the outflow catheter 46 and through the outflow passage 48a will likewise be presterilized and disposable as well as the outflow line 52. The pressure monitor 50 will preferably be designed to accept a length of presterilized tubing and sense the pressure of the fluid in the tubing without actually making contact with the fluid so that the pressure monitor may be interchanged with disposable tubing. The flow monitor 54 being of an ultrasonic type flow monitor likewise accepts a length of tubing through the flow monitor and will not ever contact the fluid. In practice, the entire set of tubing from the bacterial filter 30, through the head vessel 32, and including its liner, the tubing from the head vessel 32 to the valve 34 and including its "Y" tubing passages, and the remainder of the tubing passing through the valve 40, and to the catheter 12 will be a preformed preglued and presterilized unit. A separate preformed, presterilized and preconnected section of disposable tubing will include the tubing connected to the outflow catheter 46 passing through the flow monitor 50, the outflow passage 48a of the valve 40, and the flow monitor 54, and provided with a terminal connector which may be connected in the disposal tank 36. This will avoid the expense and time of cleaning and sterilizing valves, monitors, and fittings.

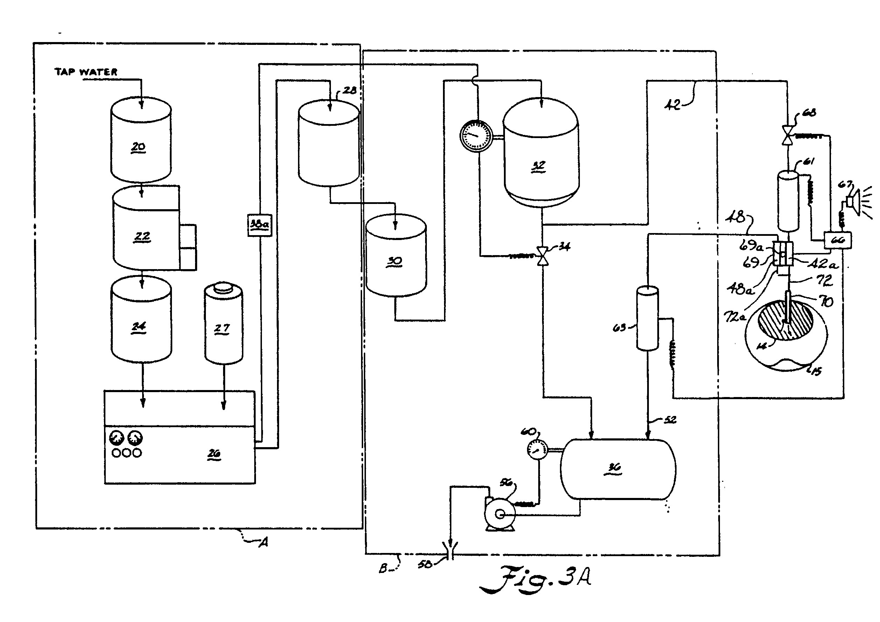

Figure 3 illustrates an alternate embodiment wherein means for maintaining a prescribed volume of fluid in the peritoneal cavity is provided by fluid volume monitors, rather than fluid pressure monitors as illustrated in Figure 2, which sense a function of the volume of fluid in the cavity and control volume in accordance with differential flow in and out of the cavity.

In the process and system of Figure 3, there is an ultrasonic flow monitor 61 disposed in the infeed line 42 for monitoring the flow of fluid in the inflow catheter 44. A second ultrasonic flow monitor 63 is disposed in the outflow line 4K connected to the outflow catheter 46. The outflow drainline 52 flows from the flow monitor 63 to the drain tank 36. There is a conventional two-way flow control valve 64 connected in the outflow drainline 52. A second conventional control valve 68 is disposed in the infeed line 42 upstream of the flow monitor 61. The monitors 61 and 63 thus sense a function of the volume of fluid in the cavity.

The control valves 64 and 68 may be controlled by any suitable process controller 66 in a conventional manner as is well within the skill of one in the art. There is an ultrasonic sensor 62 for sensing the level of fluid and hence the distension of the peritoneal cavity 14. The sensor 62 provides a second means for monitoring fluid volume, in particular dangerous levels of fluid volume. There is an alarm 67 connected to the controller 66. In the event that the volume of fluid level should increase to the dangerous amount, an audible alarm is emitted.

In use, the flow monitor 61 senses the amount of fluid flowing into the peritoneal cavity 14, and the outflow monitor 63 senses the amount of outflow fluid leaving the peritoneal cavity 14. These amounts are fed to the controller 66. The controller 66 compares the amounts of fluid flowing in the respective flow monitors and controls the valves 64, 68 to maintain a prescribed volume of fluid in the peritoneal cavity during dialysis. The prescribed amount, of course, can be varied in a conventional manner by input to the controller. The prescribed volume of fluid maintained in the peritoneal cavity 14 keeps the peritoneal cavity properly distended during dialysis. If the difference in flow between the flow monitors 61 and 63 indicates that the volume of fluid in the peritoneal cavity 14 is below the prescribed amount, the valve 64 will be closed. If the difference in volumetric flows between the flow monitors 61 and 63 indicates that too much fluid is being delivered to the peritoneal cavity 14, the valve 68 will be cut off. It is also possible that throttling valves may be utilized at 68 and 64 to control the flow in a more continuous manner.

The ultrasonic level monitor 62 is disposed appropriately in the peritoneal cavity 14. If the volume of fluid in the peritoneal cavity 14 should drop below the prescribed volume as sensed by the monitor 62, the controller 66 closes off the valve 64 to build up the volume of fluid in the peritoneal cavity 14. If the ultrasonic monitor 62 senses too large an amount of dialysis fluid in the peritoneal cavity 14, the alarm circuit 67 is triggered to produce an audible alarm and the valve 68 is closed to prevent any further fluid delivery. In the modified embodiment described above, the head vessel monitor 38 operates as described previously to control the valve 34 in a manner such that the level of fluid in the vessel 32 is maintained at a desired level.

Figure 3A illustrates a still further embodiment of the invention wherein a single catheter 70 is utilized in place of the double catheter illustrated in Figures 2 and 3 in a continuous cyclic flow. The inflow line 42 coming from the flow monitor 61 enters an inflow passage 42a of a directional valve 69. The directional valve 69 may be identical to the directional valve 40 illustrated in Figure 2. The inflow passage 42a is connected to an outflow passage 72 which is connected directly to the catheter 70. There is a parallel line 72a which is connected to the passage 72 and to an outflow passage 48a of the directional valve 69. A needle 69a of the directional valve 69 may be used to occlude and close either the passage 42a or the passage 48a selectively. The remaining system is essentially the same as Figure 3 except that the valve 64 in Figure 3 is no longer needed since the directional valve 69 controls the outflow from the catheter 70 through the outflow passage 48a.

In operation, the inflow and outflow from the peritoneal cavity 14 will both feed through the catheter 70. The directional valve 69 will control the inflow and outflow from the catheter by closing one passage while opening the other. The directional valve 69 will be controlled by a controller 66 which may be a conventional microprocessor. The flow monitors 61 and 63 have inputs connected to the controller 66. When a prescribed amount of fluid has flowed into the peritoneal cavity and abdomen, as sensed by the flow monitor 61, the directional valve 69 will be controlled to close the inflow passag 42a and open the outflow passage 48a. When the prescribed amount of fluid has flowed out of the abdomen, as sensed by the flow monitor 63, the directional valve 69 will be controlled to close the outflow passage 48a and open the inflow passage 42a. In this continuous but cyclic manner, the dialysis fluid from the continuous source A, as delivered to the head vessel 32, will be delivered through the catheter 70. Sufficient time will exist for the dialysis fluid in the peritoneal cavity to remove the water soluble metabolites. The flow will be continuous and cyclic through the single-pass open circuit from the head vessel 32, to peritoneal cavity 14, and to disposal 36.

In this way, there is no extended dwell time of the dialysis fluid in the peritoneal cavity as is occasioned by the prior "batch" methods. There is always a flow taking place either into or out of the peritoneal cavity.

**Claims**

1.  Peritoneal dialysis apparatus comprising:
    a source (32) of sterile dialysis fluid;
    an inflow line (42) for connection to the source (32) and to a peritoneal catheter (12,70) implantable in the peritoneal cavity (14) of a patient; and an outflow line (48) for connection to the peritoneal catheter (12, 70); whereby a generally continuous flow of sterile dialysis fluid is produced and caused to flow through the peritoneal cavity;
    characterised by valve means (40,68,64,69) for controlling the flow of dialysis fluid in the inflow and outflow lines thereby to control the volume of dialysis fluid in the peritoneal cavity of the patient;
    disposal means (36) connected to the outflow line for disposing of the outflow of dialysis fluid and establishing a single-pass open circuit through the peritoneal cavity for dialysis;
    flow monitoring means (61,63) for sensing a function of the volume and amount of dialysis fluid in the peritoneal cavity; and
    control means controlling the valve means in response to the flow monitoring means so that flow of the dialysis fluid through the peritoneal cavity is controlled in a manner such that a desired amount of fluid exists in the peritoneal cavity for proper dialysis.

2.  Apparatus according to claim 1, wherein the peritoneal catheter includes a double catheter (12) having an inflow catheter (44) connected to the inflow line (42) and an outflow catheter (46) connected to the outflow line (48).

3.  Apparatus according to claim 2, wherein the flow monitoring means comprises pressure monitoring means (50) in the outflow line (48) for sensing the pressure of dialysis fluid in the peritoneal cavity; and the control means controls the valve means (40) in response to the pressure monitoring means so that flow of the dialysis fluid through the peritoneal cavity is controlled to maintain a desired pressure of fluid in the cavity.

4.  Apparatus according to claim 2, including a bypass valve (34) connected between the source (32) and the valve means (40) and between the source (32) and the disposal means (36), and means controlling the bypass valve (34) to adjust the flow of sterile dialysis fluid delivered to the valve means (40).

5.  Apparatus according to claim 2, wherein the flow monitor means includes a monitor (54) for sensing the outflow of dialysis fluid from the outflow catheter (48) and controlling the valve means (40) so that the inflow and outflow of dialysis fluid are equal.

6.  Apparatus according to claim 2, wherein the flow monitor means includes a flow monitor (61, 63) in each of the inflow (42) and outflow (48) lines for sensing the flow through the peritoneal cavity and the control means controls the valve means (68,64) and the inflow and outflow of dialysis fluid to maintain a desired volume of fluid in the peritoneal cavity.

7.  Apparatus according to claim 2, wherein the valve means includes:
    an inflow/outflow valve (40) having an inflow passage (42a) connected to the inflow line and an outflow passage (48a) connected to the outflow line (48); a valve element (43) for selectively occluding either said inflow passage (42a) or the outflow passage (48a); and the flow monitoring means comprises a pressure monitor (50) for sensing the fluid pressure in the peritoneal cavity and controlling the inflow/outflow valve to occlude either the inflow (42a) or the outflow (48a) passage to regulate the pressure in the peritoneal cavity within a desired range.

8. Apparatus according to claim 7, wherein the flow control means further includes a flow monitor (54) for monitoring the outflow of the dialysis fluid from the inflow/outflow valve (40) and controlling the inflow of dialysis to the valve (40) when both the inflow and outflow passages are open so that the inflow of the dialysis fluid delivered through the valve to the peritoneal cavity and the outflow of dialysis fluid from the cavity are equal.

9. Apparatus according to claim 2, wherein: the source includes a head vessel (32) in which the sterilized dialysis fluid is stored: a bypass valve (34) is connected between the head vessel (32) and the valve means (40); a drain line connects the bypass valve (34) to a disposal means (36); and flow level sensing means (38) communicate with an interior of the head vessel (32) and control the by pass valve (34) to bypass the dialysis fluid from the head vessel (32) to the disposal means (36) should the level of the dialysis fluid reach a predetermined height in the head vessel (36).

10. Apparatus according to claim 1, including fluid level sensing means (62) for sensing the amount of dialysis fluid in the cavity and producing an alarm signal (67) in the event overfilling and overdistension of the cavity occurs.

11. Apparatus according to claim 1, wherein the inflow lines and outflow lines are connected to the valve means (69); and the valve means (69) selectively opens and closes the inflow and outflow lines to deliver a prescribed amount of dialysis fluid through the peritoneal cavity in a continuous cyclic manner.

12. Apparatus according to claim 1, wherein the valve means includes: an inflow/outflow valve (69) having an inflow passage (42a) connected to the inflow line (42) and an outflow passage (48a) connected to the outflow line (72); a valve element (69a) for selectively occluding either the inflow passage or the outflow passage; and the flow monitoring means comprises a flow monitor (61,63) in each of the inflow and outflow lines for sensing the fluid flow in the flow lines and controlling the inflow/outflow valve (69) to occlude either the inflow or the outflow passage in a manner such that a prescribed amount of dialysis fluid is delivered into the peritoneal cavity and then removed through the outflow passage (72) and line (48a) in a continuous cyclic manner.

**Revendications**

1. Appareil de dialyse péritonéale comportant: une source (32) de fluide de dialyse stérile; une canalisation d'admission (42) en vue d'un raccordement à la source (32) et à un cathéter péritonéal (12,70) pouvant être implanté dans la cavité péritonéale (14) d'un malade; et une canalisation de sortie (48) en vue d'un raccordement au cathéter péritonéal (12,70); si bien qu'un écoulement généralement continu de fluide de dialyse stérile est provoqué et amené à circuler à travers la cavité péritonéale; caractérisé par des soupapes (40,68,64,69) pour commander l'écoulement de fluide de dialyse dans les canalisations d'admission et de sortie pour ainsi contrôler le volume de fluide de dialyse dans la cavité péritonéale du malade; des moyens d'évacuation (36) reliés à la canalisation de sortie pour un rejet du fluide de dialyse et pour établir un circuit ouvert à un seul passage à travers la cavité péritonéale en vue d'une dialyse; des moyens de contrôle d'écoulement (61,63) pour détecter une fonction du volume et d'une quantité de fluide de dialyse dans la cavité péritonéale; et des moyens de commande commandant les soupapes en réponse aux moyens de contrôle d'écoulement de sorte qu'un écoulement du fluide de dialyse à travers la cavité péritonéale soit contrôlé d'une manière telle qu'une quantité voulue de fluide se trouve dans la cavité péritonéale en vue d'une dialyse correcte.

2. Appareil selon la revendication 1, dans lequel le cathéter péritonéal comprend un cathéter double (12) possédant un cathéter d'admission (44) relié à la canalisation d'admission (42) et un cathéter de sortie (46) relié à la canalisation de sortie (48).

3. Appareil selon la revendication 2, dans lequel les moyens de contrôle d'écoulement comprennent des moyens de contrôle de pression (50) dans la canalisation de sortie (48) pour détecter la pression de fluide de dialyse dans la cavité péritonéale;

les moyens de commande commandent la soupape (40) en réponse aux moyens de contrôle de pression de sorte qu'un écoulement du fluide de dialyse à travers la cavité péritonéale est commandé pour maintenir une pression voulue de fluide dans la cavité.

4. Appareil selon la revendication 2, comprenant une soupape de dérivation (34) montée entre la source (32) et la soupape (40) et entre la source (32) et les moyens de rejet (36), et des moyens commandant la soupape de dérivation (34) pour régler l'écoulement de fluide de dialyse stérile délivré à la soupape (40).

5. Appareil selon la revendication 2, dans lequel les moyens de contrôle d'écoulement comprennent un contrôleur (54) pour détecter la sortie de fluide de dialyse du cathéter de sortie (48) et commander la soupape (40) de sorte que l'admission et la sortie de fluide de dialyse soient égales.

6. Appareil selon la revendication 2, dans lequel les moyens de contrôle d'écoulement comprennent un contrôleur d'écoulement (61,63) dans chacune des canalisations d'entrée (42) et de sortie (48) pour détecter l'écoulement à travers la cavité péritonéale et les moyens de commande commandent les soupapes (68,64) et l'admission et la sortie de fluide de dialyse pour maintenir un volume désiré de fluide dans la cavité péritonéale.

7. Appareil selon la revendication 2, dans lequel les soupapes comprennent: une soupape d'admission/sortie (40) ayant un passage d'admission (42a) relié à la canalisation d'admission et un passage de sortie (48a) relié à la canalisation de sortie (48); un élément de soupape (43) pour obturer sélectivement, soit ledit passage d'admission (42a), soit le passage de sortie (48a); et les moyens de contrôle d'écoulement comprennent un contrôleur de pression (50) pour détecter la pression de fluide dans la cavité péritonéale et commander la soupape d'admission/sortie pour obturer, soit le passage d'admission (42a), soit le passage de sortie (48a) pour réguler la pression dans la cavité péritonéale à l'intérieur d'une plage désirée.

8. Appareil selon la revendication 7, dans lequel les moyens de commande d'écoulement comprennent en outre un contrôleur d'écoulement (54) pour contrôler la sortie du fluide de dialyse de la soupape d'entrée/sortie (40) et commander l'admission de fluide de dialyse vers la soupape (40) lorsque les deux passages d'admission et de sortie sont ouverts de sorte que l'admission du fluide de dialyse délivré par l'intermédiaire de la soupape à la cavité péritonéale et la sortie du fluide de dialyse de la cavité sont égales.

9. Appareil selon la revendication 2, dans lequel:
la source comprend un récipient supérieur (32) dans lequel est stocké le fluide de dialyse stérilisé;
une soupape de dérivation (34) est montée entre le récipient supérieur (32) et la soupape (40);
une canalisation de drainage relie la soupape de dérivation (34) à un Moyen de rejet (36); et des moyens de détection de niveau d'écoulement (38) communiquent avec l'intérieur du récipient supérieur (32) et commandent la soupape de dérivation (34) pour dériver le fluide de dialyse depuis le récipient supérieur (32) vers le moyen de rejet (36) au cas où le niveau du fluide de dialyse atteint une hauteur prédéterminée dans le récipient supérieur (32).

10. Appareil selon la revendication 1, comprenant des moyens de détection de niveau de fluide (62) pour détecter la quantité de fluide de dialyse dans la cavité et délivrer un signal d'alarme (67) dans le cas où un remplissage exagéré et une surdistension de la cavité se produisent.

11. Appareil selon la revendication 1, dans lequel les canalisations d'admission et les canalisations de sortie sont reliées à la soupape (69); et la soupape (69) ouvre et ferme sélectivement les canalisations d'admission et de sortie pour délivrer une quantité prescrite de fluide de dialyse à travers la cavité péritonéale d'une manière cyclique continue.

12. Appareil selon la revendication 1, dans lequel les soupapes comprennent :
une soupape d'admission/sortie (69) possédant un passage d'admission (42a) relié à la canalisation d'admission (42) et un passage de sortie (48a) relié à la canalisation de sortie (72);
un élément de soupape (69a) pour obturer sélectivement, soit le passage d'admission, soit le passage de sortie; et les moyens de contrôle d'écoulement comprennent un contrôleur d'écoulement (61,63) dans chacune des canalisations d'admission et de sortie pour détecter l'écoulement de fluide dans les canalisations d'écoulement et commander la soupape d'admission/sortie (69) pour obturer, soit le passage d'admission, soit le passage de sortie d'une manière telle qu'une quantité prescrite de fluide de dialyse soit délivrée dans la cavité péritonéale et ensuite éliminée par l'intermédiaire du passage de sortie (72) et de la canalisation (48a) d'une manière cyclique continue.

**Patentansprüche**

1. Vorrichtung zur peritonealen Dialyse mit einer Quelle (32) für sterile Dialyseflüssigkeit, einer Einlaufleitung (42) für eine Verbindung mit der Quelle (32) und einem Peritonealkatheter (12, 70), der in den Peritonealraum (14) eines Patienten einpflanzbar ist, und einer Auslaufleitung (48) zur Verbindung mit dem Peritonealkatheter (12,70), wobei ein allgemein kontinuierlicher Fluß von steriler Dialyseflüssigkeit erzeugt und zum Fließen durch den Peritonealraum gebracht wird, **gekennzeichnet durch** Ventileinrichtungen (40, 68, 64, 69) zur Steuerung des Flusses der Dialyseflüssigkeit in den Einlauf- und Auslaufleitungen und damit zur Steuerung des Dialyseflüssigkeitsvolumens in dem Peritonealraum des Patienten, eine mit der Auslaufleitung verbundene Entsorgungseinrichtung (36) zur Entsorgung des Auslaufs von Dialyseflüssigkeit und zur Herstellung eines offenen Einwegkreislaufs durch den Peritonealraum für die Dialyse, Flußüberwachungseinrichtungen (61, 63) zum Abfühlen einer Funktion des Volumens und der Menge der Dialyseflüssigkeit in dem Peritonealraum und Steuereinrichtungen, die die Ventileinrichtungen in Abhängigkeit von den Flußüberwachungseinrichtungen derart steuern, daß der Fluß der Dialyseflüssigkeit durch den Peritonealraum so gesteuert wird, daß eine für ausreichende Dialyse erwünschte Flüssigkeitsmenge in dem Peritonealraum vorliegt.

2. Vorrichtung nach Anspruch 1, bei der der Peritonealkatheter einen Doppelkatheter (12) mit einem Einlaufkatheter (44), der mit der Einlaufleitung (42) verbunden ist, und einem Auslaufkatheter (46), der mit der Auslaufleitung (48) verbunden ist, enthält.

3. Vorrichtung nach Anspruch 2, bei der die Flußülberwachungseinrichtungen eine Drucküberwachungseinrichtung (50) in der Auslaufleitung (48) zum Anfühlen des Druckes der Dialyseflüssigkeit in dem Peritonealraum umfaßt und daß die Steuereinrichtung die Ventileinrichtungen (40) in Abhängigkeit von der Drucküberwachungseinrichtung derart steuert, daß der Fluß der Dialyseflüssigkeit durch den Peritonealraum so gesteuert wird, daß in dem Raum ein erwünschter Flüssigkeitsdruck aufrechterhalten wird.

4. Vorrichtung nach Anspruch 2 mit einem Bypass-Ventil (34), das zwischen der Quelle (32) und der Ventileinrichtung (40) und zwischen der Quelle (32) und der Entsorgungseinrichtung (36) eingeschaltet ist, und mit das Bypass-Ventil (34) steuernden Einrichtungen, die den Fluß der sterilen Dialyseflüssigkeit, die an die Ventileinrichtung (40) geliefert wird, einstellen.

5. Vorrichtung nach Anspruch 2, bei der die Flußüberwachungseinrichtung eine Überwachungseinrichtung (54) zum Anfühlen des Auslaufs der Dialyseflüssigkeit aus dem Auslaufkatheter (48) und zur Steuerung der Ventileinrichtung (40) derart, daß der Einlauf und der Auslauf der Dialyseflüssigkeit gleich sind, enthält.

6. Vorrichtung nach Anspruch 2, bei der die Flußüberwachungseinrichtung eine Flußüberwachungsvorrichtung (61, 63) jeweils in der Einlaufleitung (42) und der Auslaufleitung (48) zum Anfühlen des Flusses durch den Peritonealraum enthält und daß die Steuereinrichtung die Ventileinrichtungen (68, 64) und den Einlauf und Auslauf der Dialyseflüssigkeit steuert, um das erwünschte Flüssigkeitsvolumen in dem Peritonealraum aufrechtzuerhalten.

7. Vorrichtung nach Anspruch 2, bei der die Ventileinrichtungen ein Einlauf/Auslauf-Ventil (40) mit einem mit der Einlaufleitung verbundenen Einlaufdurchgang (42a) und einem mit der Auslaufleitung (48) verbundenen Auslaufdurchgang (48a) und ein Ventilelement (43) für selektives Ansperren entweder des Einlaufdurchganges (42a) oder des Auslaufdurchganges (48a) enthält und die Flußüberwachungseinrichtung eine Drucküberwachungsvorrichtung (50) zum Anfühlen des Flüssigkeitsdruckes in dem Peritonealraum und zur Steuerung des Einlauf/Auslauf-Ventils aufweist, um entweder den Einlaufdurchgang (42a) oder den Auslaufdurchgang (48a) abzusperren und so den Druck in dem Peritonealraum in einem erwünschten Bereich zu regulieren.

8. Vorrichtung nach Anspruch 7, bei der die Flußsteuereinrichtung außerdem eine Flußüberwachungsvorrichtung (54) zum Überwachen des Auslaufs der Dialyseflüssigkeit aus dem Einlauf/Auslauf-Ventil (40) und zur Steuerung des Dialyseeinlaufes zu dem Ventil (40), wenn sowohl der Einlaufdurchgang als auch der Auslaufdurchgang offen sind, aufweist, so daß der Einlauf der Dialyseflüssigkeit, die durch das Ventil zu dem Peritonealraum geht, und der Auslauf der Dialyseflüssigkeit aus dem Raum gleich sind.

9. Vorrichtung nach Anspruch 2, bei der die Quelle einen Kopfbehälter (32), in welchem die sterilisierte Dialyseflüssigkeit gelagert wird, enthält, ein Bypass-Ventil (34) zwischen dem Kopfbehälter (32) und der Ventileinrichtung (40) eingeschaltet ist, eine Abschlußleitung das BypassVentil (34) mit einer Entsorgungseinrichtung (36) verbindet und eine Flußmengenabfühleinrichtung (38) mit dem Inneren des Kopfbehälters (32) verbunden ist und das Bypass-Ventil (34) so steuert, daß die Dialyseflüssigkeit von dem Kopfbehälter (32) zu der Entsorgungseinrichtung (36) umgelenkt wird, wenn die Menge der Dialyseflüssigkeit eine vorbestimmte Höhe in dem Kopfbehälter (36) erreicht.

10. Vorrichtung nach Anspruch 1 mit einer Flüssigkeitsmengenabfühleinrichtung (62) zum Anfühlen der Menge der Dialyseflüssigkeit im Peritonealraum und zur Erzeugung eines Alarmsignals (67) im Falle, daß eine Überfüllung und Überdehnung des Peritonealraumes auftritt.

11. Vorrichtung nach Anspruch 1, bei der die Einlaufleitungen und Auslaufleitungen mit der Ventileinrichtung (69) verbunden sind und die Ventileinrichtung (69) selektiv die Einlauf- und Auslaufleitungen öffnet und schließt, um eine vorbestimmte Menge der Dialyseflüssigkeit durch den Peritonealraum in einer kontinuierlichen zyklischen Weise zu befördern.

12. Vorrichtung nach Anspruch 1, bei der die Ventileinrichtung ein Einlauf/Auslauf-Ventil (69) mit einem mit der Einlaufleitung (42) verbundenen Einlaufdurchgang (42a) und einem mit der Auslaufleitung (72) verbundenen Auslaufdurchgang (48a) und ein Ventilelement (69a) für selektives Absperren entweder des Einlaufdurchgangs oder des Auslaufdurchgangs enthält und die Flußüberwachungseinrichtung eine Flußüberwachungsvorrichtung (61, 63) jeweils in der Einlauf-und Auslaufleitung zum Abfühlen des Flüssigkeitsflusses in den Fließleitungen und zur Steuerung des Einlauf/AuslaufVentils (69) aufweist, um entweder den Einlaufdurchgang oder den Auslaufdurchgang in solcher Weise abzusperren, daß in kontinuierlicher zyklischer Weise eine vorbestimmte Menge der Dialyseflüssigkeit in den Peritonealraum geliefert und dann durch den Auslaufdurchgang (72) und die Auslaufleitung (48a) entfernt wird.

*Fig.1*

Fig.2

Fig.3

TAP WATER

Fig. 3A